(19) Europäisches Patentamt
European Patent Office
Office européen des brevets

(11) Numéro de publication: **0 115 887**
**B1**

(12) **FASCICULE DE BREVET EUROPEEN**

(45) Date de publication du fascicule du brevet: **01.04.87**

(51) Int. Cl.[4]: **C 07 C 29/64**, C 07 C 31/40

(21) Numéro de dépôt: **84200078.8**

(22) Date de dépôt: **23.01.84**

(54) **Procédé pour l'obtention du 2,2,2-trifluoroéthanol.**

(30) Priorité: **02.02.83 FR 8301837**

(43) Date de publication de la demande:
**15.08.84 Bulletin 84/33**

(45) Mention de la délivrance du brevet:
**01.04.87 Bulletin 87/14**

(84) Etats contractants désignés:
**AT BE CH DE FR GB IT LI LU NL SE**

(56) Documents cités:
**US - A - 3 146 274**
**US - A - 3 367 982**

**CHEMICAL ABSTRACTS, vol. 69, no. 5, 29 juillet 1968, page 1726, no. 18477v, Columbus, Ohio, US G.G. BELEN'KII et al.: "Potassium permanganate in hypofluorination reactions"**
**CHEMICAL ABSTRACTS, vol. 64, no. 9, 25 avril 1966, colonne 12534, no. c, Columbus, Ohio, US L.S. GERMAN et al.: "Hypofluorination reaction"**
**CHEMICAL ABSTRACTS, vol. 66, no. 15, 10 avril 1967, page 6093, no. 64988y, Columbus, Ohio, US L.S. GERMAN et al.: "Hydrogen peroxide in the hypoflurination reaction"**

(73) Titulaire: **SOLVAY & Cie (Société Anonyme), Rue du Prince Albert, 33, B-1050 Bruxelles (BE)**

(72) Inventeur: **Lecloux, André, Van Immersseellaan 15, B-1860 Meise (BE)**
Inventeur: **Legrand, Franz, Rue des Vaches 71, B-7300 Quaregnon (BE)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé pour l'obtention du 2,2,2-trifluoroéthanol à partir de 1,1-difluoroéthylène et d'un oxydant tel que l'oxygène.

Il est connu par les publications soviétiques Izv. Akad. Nauk., SSSR, Ser Khim, 1968 (3) pages 554-558 et Dokl. Akad. Nauk., SSSR, 1966, 166 (3) pages 602-603 résumées respectivement dans Chemical Abstracts 1968, 63 n° 18477v et Chemical Abstracts 1966, 64, page 12534 (c) que des 2,2,2-trihalogénoéthanols, répondant à la formule

$CX_1X_2F-CH_2OH$

dans laquelle $X_1$ et $X_2$ sont identiques ou différents et représentent le fluor ou le chlore, peuvent être obtenus par hypofluoration en présence d'un oxydant des 1,1-dihalogénoéthylènes de formule $CX_1X_2{=}CH_2$. Ces publications enseignent que pour obtenir en particulier du 2,2,2-trifluoroéthanol, on additionne lentement et selon des proportions bien définies du 1,1-difluoroéthylène à un mélange maintenu à −78°C contenant en proportions également bien définies de l'acide fluorhydrique anhydre, de l'éther absolu et du trioxyde de chrome ($CrO_3$). L'emploi de permanganate de potassium ($KMnO_4$) comme oxydant y est également enseigné.

Ce procédé d'obtention des 2,2,2-trihalogénoéthanols $CX_1X_2F-CH_2OH$ et notamment du 2,2,2-trifluoréthanol pose de sérieuses difficultés lorsqu'on envisage son exploitation industrielle. En effet, il y est fait usage d'acide fluorhydrique anhydre, ce qui impose de recourir à des matériaux coûteux résistant à la corrosion et il est nécessaire d'opérer à très basse température.

La présente invention permet d'éviter les inconvénients précités et fournit un procédé permettant d'opérer selon des conditions plus économiques et dans des matériaux habituellement mis en oeuvre au stade industriel. De plus, l'invention fournit un procédé exploitable en continu.

L'invention concerne à cet effet un procédé pour l'obtention de 2,2,2-trifluoroéthanol à partir de 1,1-difluoroéthylène en phase gazeuse entre 150 et 350°C avec de l'oxygène moléculaire et un catalyseur d'oxydation choisi parmi les métaux ou les dérivés des métaux des groupes Ib, IIb, IIIb, IVb, Vb, VIb, VIIb et VIII du tableau périodique des éléments de la classification internationale des éléments (Handbook of Chemistry and Physics, 51st edition, 1970-1971.

De préférence on utilise les métaux des Groupes Ib, IIb, IIIb, IVb et Vb ou les oxydes correspondants. On a obtenu les meilleurs résultats en utilisant l'argent comme catalyseur d'oxydation.

Les catalyseurs d'oxydation selon l'invention peuvent être utilisés tels quels ou associés à d'autres matériaux agissant comme supports. Généralement, on les utilise en association avec un support. Dans ce cas, les supports sont choisis de manière à conférer aux catalyseurs d'oxydation l'amélioration d'une ou de plusieurs de leurs caractéristiques telles que la résistance mécanique, la dimension des particules et leur distribution, le volume poreux, la surface spécifique, l'activité catalytique, la productivité catalytique, la sélectivité.

A cet effet, tous les matériaux couramment utilisés comme supports de catalyseurs peuvent être utilisés, c'est-à-dire par exemple les métaux, les oxydes de métaux, les dérivés oxydés des métalloïdes et de terres rares, les terres de diatomées, la pierre ponce, les bentonites, les charbons actifs, les gels de silice. De préférence, les supports sont choisis parmi les oxydes des éléments des Groupes IIa, IIIa et IVa du tableau périodique des éléments. Des supports convenant bien sont les oxydes d'aluminium ou alumines. Parmi ceux-ci, on sélectionne avantageusement ceux ayant une surface spécifique inférieure à 5 $m^2/g$ et en particulier ceux de surface spécifique inférieure à 1 $m^2/g$. De bons résultats ont été obtenus avec des surfaces spécifiques comprises entre 0,01 et 0,5 $m^2/g$. Les supports tout particulièrement préférés sont les alumines de type alpha.

On obtient de bons résultats lorsque le support sélectionné est une alumine se présentant sous la forme de petits granules, poreux ou non, de diamètre moyen compris entre 0,005 et 5 cm. Les meilleurs résultats ont été obtenus avec des grains d'alumine de diamètre moyen compris entre 0,01 et 0,5 cm.

Le catalyseur d'oxydation peut être associé au support en toutes proportions. Lorsque l'on opère avec de l'argent supporté sur une alumine de type alpha, généralement on réalise une proportion comprise entre 0,1 et 100 g d'argent par 100 g d'alumine de type alpha. De préférence on choisit une proportion comprise entre 1 et 50 g d'argent par 100 g d'alumine de type alpha.

Dans le mode d'exécution du procédé selon l'invention qui consiste à utiliser comme catalyseur d'oxydation de l'argent supporté sur une alumine de type alpha, on a constaté que la pureté de l'alumine alpha a une grande importance. Il est donc avantageux de choisir une alumine de type alpha aussi pure que possible. De préférence, on choisit une alumine de type alpha contenant moins de 2% en poids d'impuretés et en particulier d'impuretés contenant des métaux tels que le fer. De manière avantageuse, on choisit une alumine de type alpha contenant moins de 0,6% en poids de fer par rapport au poids d'aluminium contenu dans l'alumine. De manière particulièrement avantageuse, on choisit une alumine de type alpha contenant moins de 0,2% en poids de fer par rapport à la teneur en aluminium de l'alumine.

Le catalyseur d'oxydation peut être incorporé au support selon toutes les techniques connues et notamment par imprégnation, précipitation, coprécipitation, mélange mécanique, adsorption en phase vapeur ou liquide et vaporisation. Lorsque l'on opère avec de l'argent et une alumine de type alpha, une exécution ayant donné de bons résultats consiste à d'abord imprégner l'alumine de type alpha avec une solution comprenant un solvant (tel que l'eau), un sel d'argent dissous dans ce solvant, une base azotée (telle que l'ammoniac et les amines) et un réducteur organique (tel que le formaldéhyde et certaines amines organiques), à ensuite éliminer le solvant et à enfin soumettre le solide obtenu à un traitement thermique. Les meilleurs résultats ont été obtenus lorsque l'imprégnation de l'alumine de type alpha est réalisée en présence d'agents chimiques, aussi appelés promoteurs, permettant d'améliorer la dispersion de l'argent dans l'alumine de type alpha et le maintien de cet état dispersé au cours du temps. Des exemples de tels promoteurs sont des sels ou composés basiques dérivés de métaux alcalins ou alcalino-terreux tels que l'hydroxyde de sodium ou de potassium ainsi que les acétates de ces mêmes métaux.

Dans le procédé selon l'invention, on peut opérer en présence ou non de diluants. Par diluants, on entend désigner des composés chimiquement inertes dans la réaction d'obtention du 2,2,2-trifluoroéthanol du procédé selon l'invention. Il s'agit des gaz tels que l'azote ou l'anhydride carbonique. On peut disposer le catalyseur d'oxydation en lit fluide ou en lit fixe. De bons résultats ont été obtenus en disposant le catalyseur d'oxydation en lit fixe.

Dans le procédé selon l'invention, on peut en principe faire réagir le 1,1-difluoréthylène et l'oxydant en toutes proportions. Toutefois, certains mélanges de 1,1-difluoroéthylène et d'oxydant dénommés ci-après «mélanges réactionnels» peuvent être explosifs et il convient de déterminer pour chaque composition du mélange réactionnel, les limites d'explosibilité de façon à définir des conditions d'obtention de 2,2,2-trifluoroéthanol ne présentant que peu ou pas de risque d'explosion. Ainsi lorsqu'on opère avec un mélange réactionnel comprenant du 1,1-difluoroéthylène et de l'oxygène moléculaire non dilué,on observe la formation de mélanges réactionnels explosifs dès que, aux températures et pressions décrites dans les exemples, le mélange réactionnel contient plus de 10% en volume d'oxygène moléculaire. Il est toutefois évident que les compositions explosives du mélange réactionnel sont fonction non seulement de la température et de la pression mais également de la présence ou non d'autres composés tels que les diluants cités ci-avant.

On a constaté que, dans le procédé selon l'invention, la température de réaction est fonction de la nature du catalyseur d'oxydation mis en oeuvre. Avec un catalyseur d'oxydation constitué d'argent supporté sur une alumine de type alpha, on choisit habituellement une température comprise entre 150 et 350°C. On a obtenu les meilleurs résultats lorsque la température a été choisie entre 250 et 300°C.

Dans le procédé selon l'invention, on préfère pour des raisons de productivité, appliquer une pression partielle en 1,1-difluoroéthylène aussi élevée que possible. Habituellement les pressions appliquées se situent entre la pression atmosphérique et 100 bars. Au laboratoire, on a obtenu de bons résultats quand la pression appliquée était d'environ 10 bars.

On a constaté que les catalyseurs d'oxydation selon l'invention peuvent être utilisés pendant des durées de réaction relativement longues sans que leur activité en soit sensiblement affectée, et le procédé selon l'invention peut dès lors être exploité en continu ou en discontinu.

L'appareillage pour la mise en oeuvre du procédé selon l'invention n'exige aucune exécution ni aucun matériau particulier. Tout appareillage dans lequel on peut appliquer les températures et pressions citées ci-avant permettant l'évacuation des calories exédentaires, peut convenir.

Le 2,2,2-trifluoroéthanol obtenu par le procédé selon l'invention peut être utilisé tel quel ou après séparation du mélange réactionnel et sous-produits éventuels obtenus, dans toutes les applications connues de ce produit et notamment comme solvant des polymères fluorés ou comme réactif pouvant être oxydé dans le but d'obtenir l'aldéhyde ou l'acide correspondant.

L'invention est illustrée par les exemples décrits ci-après.

Exemple 1

1. Préparation du catalyseur

Dans un ballon de 250 $cm^3$ on introduit 8 g d'acétate d'argent dissous dans 8 $cm^3$ de $NH_4OH$.13N. On ajoute à la solution, 50 mg de sodium sous forme d'acétate de soude, 0,5 mg de potassium sous forme d'hydroxyde de potassium et 1,2 ml de triéthanolamine, sous agitation. Après homogénéisation, on imprègne avec cette solution 120 g d'alumine du type alpha, contenant moins de 0,1% en poids de fer, ayant une surface spécifique de 0,3 $m^2/g$ et un diamètre moyen des granules de 0,25 mm. Ce mélange est chauffé en étuve à 100°C pendant 2 h. Ensuite, le solide récupéré est chauffé sous courant d'azote à 300°C pendant 4 h. Le catalyseur obtenu contient 4% en poids d'argent finement divisé supporté sur l'alumine de type alpha.

2. Réaction d'oxydation

5 $cm^3$ de catalyseur préparé comme décrit ci-dessus sont disposés en lit fixe dans un réacteur tubulaire constitué par un tube en acier inoxydable d'un diamètre de 3/8″ et sont chauffés à 280°C. On fait passer sur ce catalyseur en continu avec un débit de 15 $cm^3$ Normaux/min. un mélange gazeux constitué de 90% en volume de 1,1-difluoroéthylène et de 10% en volume d'oxygène moléculaire sous une pression de 8 bars. Comme signalé plus haut, la proportion du mélange 1,1-difluoroéthylène et oxygène est choisie de manière à former un mélange non explosif. A la sortie du tube les différents constituants du mélange réactionnel sont dosés directement par chromatographie en phase vapeur. Les constituants dosés au cours du temps sont repris au tableau ci-après.

Tableau

| Constituant en % vol. | Temps en heures | | | | | | | |
|---|---|---|---|---|---|---|---|---|
| | 1,5 | 3 | 4,5 | 6 | 7,5 | 9 | 10,5 | 12 |
| $O_2$ | 1,5 | 1,4 | 1,2 | 1,3 | 1,3 | 1,5 | 1,7 | 2,0 |
| 1,1-difluoroéthylène | 85,9 | 85,7 | 85,6 | 85,4 | 85,5 | 85,6 | 85,5 | 85,5 |
| 2,2,2-trifluoroéthanol | 5,9 | 6,0 | 6,1 | 6,1 | 6,1 | 5,9 | 5,7 | 5,4 |
| $CO_2$ | 7,0 | 6,9 | 7,0 | 6,9 | 6,8 | 6,6 | 6,5 | 6,2 |

Dans cet exemple, la sélectivité en trifluoroéthanol par rapport au 1,1-difluoroéthylène consommé est constante et voisine de 65%. Le $CO_2$ est le sous-produit principal.

Exemple 2

1. Préparation du catalyseur

Le catalyseur a été préparé comme décrit à l'exemple 1 excepté que les quantités de sels d'argent, de sodium et de potassium mises en oeuvre ont été doublées.

2. Réaction d'oxydation

Les conditions opératoires sont identiques à celles décrites à l'exemple 1, mais l'on fait passer en continu sur le catalyseur, avec un débit de 30 $cm^3$ N/min., un mélange gazeux constitué de (90 - X) % en volume de 1,1-difluoroéthylène, de 10% en volume d'oxygène et de X% en volume de $CO_2$, sous une pression totale de 8 bars.

Le tableau ci-dessous donne pour différents mélanges les teneurs respectives en 2,2,2-trifluoroéthanol après 3 h de réaction (ramenées à une même pression partielle initiale en 1,1-difluoroéthylène), la teneur trouvée pour le mélange exempt de $CO_2$ étant prise égale à 1.

Tableau

| X | 2,2,2-trifluoroéthanol |
|---|---|
| 0 | 1 |
| 11 | 0,98 |
| 19 | 0,95 |
| 50 | 0,95 |

Dans tous les cas, la sélectivité en 2,2,2-trifluoroéthanol par rapport au 1,1-difluoroéthylène consommé est voisine de 65%. Le $CO_2$ est le sous produit principal. Cet exemple montre que la présence d'un diluant tel que l'anhydride carbonique n'affecte pas la réaction et que la productivité est proportionnelle à la pression partielle de 1,1-difluoroéthylène.

**Revendications**

1. Procédé pour l'obtention du 2,2,2-trifluoro éthanol à partir du 1,1-difluoroéthylène à l'intervention notamment d'un oxydant, caractérisé en ce qu'on fait réagir le 1,1-difluoroéthylène en phase gazeuse entre 150° et 350° avec de l'oxygène moléculaire et un catalyseur d'oxydation choisi parmi les métaux ou les dérivés des métaux des groupes Ib, IIb, IIIb, IVb, Vb, VIb, VIIb, et VIII du tableau périodique des éléments.

2. Procédé selon la revendication 1, caractérisé en ce qu'on utilise un métal ou un oxyde de ce métal appartenant aux Groupes Ib, IIb, IIIb, IVb ou Vb du tableau périodique des éléments.

3. Procédé selon la revendication 2, caractérisé en ce qu'on utilise l'argent.

4. Procédé selon l'une quelconque des revendications 1 à 3, caractérisé en ce que le catalyseur d'oxydation est associé à un support.

5. Procédé selon la revendication 4, caractérisé en ce que le support est un oxyde d'un élément des Groupes IIa, IIIa ou IVa du tableau périodique des éléments.

6. Procédé selon la revendication 5, caractérisé en ce que le support est de l'oxyde d'aluminium de type alpha.

**Patentansprüche**

1. Verfahren zur Darstellung von 2,2,2-Trifluorethanol aus 1,1-Difluorethylen, insbesondere unter der Einwirkung eines Oxydationsmittels, dadurch gekennzeichnet, dass man 1,1-Difluorethylen in der Gasphase zwischen 150° und 350° mit molekularem Sauerstoff und einem Oxidationskatalysator, ausgewählt unter den Metallen oder den Metallderivaten der Gruppen Ib, IIb, IIIb, IVb, Vb, VIb, VIIb und VIII des Periodensystems der Elemente, reagieren lässt.

2. Verfahren nach Anspruch 1, dadurch gekennzeichnet, dass man ein Metall oder ein Oxid dieses Metalles, das zu den Gruppen Ib, IIb, IIIb, IVb oder Vb des Periodensystems der Elemente gehört, verwendet.

3. Verfahren nach Anspruch 2, dadurch gekennzeichnet, dass man Silber verwendet.

4. Verfahren nach einem der Ansprüche 1 bis 3, dadurch gekennzeichnet, dass der Oxidationskatalysator mit einem Träger verbunden wird.

5. Verfahren nach Anspruch 4, dadurch gekennzeichnet, dass der Träger ein Oxid eines Elementes der Gruppen IIa, IIIa oder IVa des Periodensystems der Elemente ist.

6. Verfahren nach Anspruch 5, dadurch gekennzeichnet,dass der Träger Aluminiumoxid des alpha-Typs ist.

**Claims**

1. Process for producing 2,2,2-trifluoroethanol

from 1,1-difluoroethylene involving, in particular, an oxidizing agent, characterized in that 1,1-difluoroethylene is reacted in the gaseous phase at between 150° and 350° with molecular oxygen and an oxidation catalyst chosen from metals or derivatives of metals of Groups Ib, IIb, IIIb, IVb, Vb, VIb, VIIb and VIII of the Periodic Table of the elements.

2. Process according to Claim 1, characterized in that a metal or an oxide of this metal belonging to Groups Ib, IIb, IIIb, IVb or Vb of the Periodic Table of the elements is used.

3. Process according to Claim 2, characterized in that silver is used.

4. Process according to any one of Claims 1 to 3, characterized in that the oxidation catalyst is in combination with a support.

5. Process according to Claim 4, characterized in that the support is an oxide of an element of Groups IIa, IIIa or IVa of the Periodic Table of the elements.

6. Process according to Claim 5, characterized in that the support is aluminium oxide of the alpha type.